# EUROPEAN PATENT APPLICATION

(11) **EP 3 332 775 A1**
(43) Date of publication of application: **13.06.2018**
(21) Application number: 16832528.0
(22) Date of filing: 03.08.2016
(51) Int. Cl.: A61K 9/48, A61K 31/167, A61K 47/10, A61K 47/14, A61K 47/32, A61K 47/38, A61K 47/42, A61P 29/00

(54) **ENTERIC CAPSULE**

(30) Priority: 04.08.2015 JP 2015154420
(71) Applicant: Fuji Capsule Co., Ltd., Fujinomiya-shi, Shizuoka 418-0112 (JP)
(72) Inventor: SHIMOKAWA, Yoshiyuki, Fujinomiya-shi Shizuoka 418-0112 (JP); HAYANO, Akihiko, Fujinomiya-shi Shizuoka 418-0112 (JP)
(74) Representative: Bryers LLP
(86) International application number: PCT/JP2016/003592
(87) International publication number: WO 2017/022248

(57) **Abstract**

There are manufactured an enteric capsule comprising: a seamless capsule consisting of a capsule fill and a shell layer; and an enteric coating layer on the shell layer, the enteric coating layer comprising one selected from a methacrylic acid-based polymer, polyvinyl acetate phthalate, organic acid ester of hydroxypropylmethylcellulose, carboxymethylethylcellulose, and cellulose acetate phthalate; and an enteric seamless capsule further comprising a subcoating layer between the shell layer of the seamless capsule and the enteric coating layer, the subcoating layer consisting of hydroxypropylcellulose and the like. The enteric seamless capsule has acid resistance and also enables controlled dissolution for each application site in the body.

## Description

### Technical Field

The present invention relates to an enteric capsule, and more specifically, relates to an enteric capsule comprising: a seamless capsule consisting of a capsule fill and a shell layer; and an enteric coating layer on the shell layer, the enteric coating layer comprising one or more selected from the group consisting of a methacrylic acid-based polymer, polyvinyl acetate phthalate, organic acid ester of hydroxypropyl methylcellulose, carboxymethylethylcellulose, and cellulose acetate phthalate; and to an enteric capsule comprising: a seamless capsule consisting of a capsule fill and a shell layer; and a subcoating layer and the enteric coating layer on the shell layer.

### Background Art

A drug which is inactivated or deteriorated in efficacy by gastric juices, a drug that stimulates the gastric mucosa, and a drug that inhibits process of digestion in the stomach are encapsulated in an enteric capsule to prescribe. As the enteric capsule, a capsule has been known that is prepared from a composition comprising an enteric substance and agar wherein hydroxypropyl methylcellulose phthalate, a copolymer of methacrylic acid and alkyl methacrylate (also referred to as methacrylic acid-based polymer), ammonium salt or alkali metal salt of cellulose acetate phthalate, or the like is used as the enteric substance (Patent Document 1).

There also has been known an enteric capsule obtained by coating a seamless capsule with an enteric substance. For example, there have been known a capsule obtained by coating with an enteric substance a seamless capsule in which useful intestinal bacteria that is inactivated by gastric juices is encapsulated (Patent Document 2), and a capsule obtained by coating with an enteric substance a seamless capsule in which an immunogen is encapsulated (Patent Document 3). However, the enteric substance used for the coatings of these capsules is zein or hydroxymethylcellulose, and it is not known to use a methacrylic acid-based polymer, polyvinyl acetate phthalate, organic acid ester of hydroxypropylmethylcellulose, carboxymethylethylcellulose, or cellulose acetate phthalate as an enteric substance.

Further, there has been known a capsule obtained by coating a seamless capsule comprising medicine solution or dispersion with an enteric substance for the purpose of improving the oral absorbability of not only useful intestinal bacteria and immunogens but also medicines having a low absorbability in the digestive tract (Patent Document 4). The capsule has a three layer structure, i.e., a structure consisting of a capsule fill, a hydrophobic layer consisting of hydrogenated oil and lecithin, and a shell layer. The document does not disclose a capsule obtained by coating a seamless capsule having a two layer structure consisting of a capsule fill and a shell layer with a methacrylic acid-based polymer, polyvinyl acetate phthalate, organic acid ester of hydroxypropylmethylcellulose, carboxymethylethylcellulose, or cellulose acetate phthalate as an enteric substance.

### Prior Art Documents

### Patent Documents

Patent Document 1: Japanese unexamined Patent Application Publication No. S57-32230
Patent Document 2: Japanese unexamined Patent Application Publication No. S62-201823
Patent Document 3: Japanese unexamined Patent Application Publication No. H5-294845
Patent Document 4: Japanese examined Patent Publication No. 4860164

### Summary of the Invention

### Object to be Solved by the Invention

An object of the present invention is to obtain an enteric seamless capsule having acid resistance. Another object of the present invention is to obtain an enteric seamless capsule that enables controlled dissolution for each application site in the body. Another object of the present invention is to obtain an enteric seamless capsule that enables controlled dissolution for each application site in the body.

### Means to Solve the Object

A capsule has enteric properties and enables controlled dissolution for each application site in the body in which the capsule comprises: a seamless capsule consisting of a capsule fill and a shell layer; and a coating layer on the shell layer, the coating layer comprising one or more selected from the group consisting of a methacrylic acid-based polymer, polyvinyl acetate phthalate, organic acid ester of hydroxypropylmethylcellulose, carboxymethylethylcellulose, and cellulose acetate phthalate.

Specifically, the present invention relates to the following.
(1) An enteric capsule comprising:
   a seamless capsule consisting of a capsule fill and a shell layer; and
   an enteric coating layer on the shell layer, the enteric coating layer comprising one or more selected from the group consisting of a methacrylic acid-based polymer, polyvinyl acetate phthalate, organic acid ester of hydroxypropylmethylcellulose, carboxymethylethylcellulose, and cellulose acetate phthalate.
(2) The enteric capsule according to (1), wherein the shell layer of the seamless capsule comprises gelatin and glycerin.
(3) The enteric capsule according to (1) or (2), wherein the enteric coating layer comprises 30 to 80 parts by mass of one or more selected from the group consisting of a methacrylic acid-based polymer, polyvinyl acetate phthalate, and cellulose acetate phthalate based on 100 parts by mass of the enteric coating layer.
(4) The enteric capsule according to (1) or (2), wherein the enteric coating layer comprises 50 to 100 parts by mass of one or more selected from the group consisting of an organic acid ester of hydroxypropylmethylcellulose, carboxymethylethylcellulose, and cellulose acetate phthalate based on 100 parts by mass of the enteric coating layer.
(5) The enteric capsule according to (1) or (2), wherein the enteric coating layer comprises 30 to 80 parts by mass of one or more selected from the group consisting of a methacrylic acid-based polymer, polyvinyl acetate phthalate, and cellulose acetate phthalate and 0 to 10 parts by mass of triethyl citrate based on 100 parts by mass of the enteric coating layer.
(6) The enteric capsule according to (1) or (2), wherein the enteric coating layer comprises 50 to 100 parts by mass of one or more selected from the group consisting of organic acid ester of hydroxypropylmethylcellulose, carboxymethylethylcellulose, and cellulose acetate phthalate and 0 to 10 parts by mass of triethyl citrate based on 100 parts by mass of the enteric coating layer.
(7) The enteric capsule according to any one of (1) to (3) and (5), wherein the methacrylic acid-based polymer is a copolymer of methacrylic acid and ethyl acrylate and/or a copolymer of methacrylic acid and methyl methacrylate.
(8) The enteric capsule according to any one of (1), (2), (4) and (6), wherein the organic acid ester of hydroxypropylmethylcellulose is hydroxypropylmethylcellulose acetate succinate or hydroxypropylmethylcellulose phthalate.
(9) The enteric capsule according to any one of (1) to (8), further comprising a subcoating layer between the shell layer of the seamless capsule and the enteric coating layer.
(10) The enteric capsule according to (9), wherein the subcoating layer consists of hydroxypropylcellulose.
(11) A method for manufacturing an enteric capsule, comprising:
   coating a seamless capsule consisting of a capsule fill and a shell layer with a composition for an enteric coating to form an enteric coating layer, the composition comprising one or more selected from the group consisting of a methacrylic acid-based polymer, polyvinyl acetate phthalate, organic acid ester of hydroxypropylmethylcellulose, carboxymethylethylcellulose, and cellulose acetate phthalate.
(12) A method for manufacturing an enteric capsule, comprising:
   coating a seamless capsule consisting of a capsule fill and a shell layer with a composition for a subcoating to form a subcoating layer; and then,
   coating the subcoating layer with a composition for an enteric coating to form an enteric coating layer, wherein the composition comprising one or more selected from the group consisting of a methacrylic acid-based polymer, polyvinyl acetate phthalate, organic acid ester of hydroxypropylmethylcellulose, carboxymethylethylcellulose, and cellulose acetate phthalate.
(13) The method according to (11) or (12), wherein the composition for an enteric coating comprises 30 to 80 parts by mass of one or more selected from the group consisting of a methacrylic acid-based polymer, polyvinyl acetate phthalate, and cellulose acetate phthalate based on 100 parts by mass of the enteric coating layer formed from the composition for an enteric coating.
(14) The method according to (11) or (12), wherein the composition for an enteric coating comprises 50 to 100 parts by mass of one or more selected from the group consisting of organic acid ester of hydroxypropylmethylcellulose, carboxymethylethylcellulose, and cellulose acetate phthalate based on 100 parts by mass of the enteric coating layer formed from the composition for an enteric coating.
(15) The method according to (11) or (12), wherein the composition for an enteric coating comprises 30 to 80 parts by mass of one or more selected from the group consisting of a methacrylic acid-based polymer, polyvinyl acetate phthalate, and cellulose acetate phthalate and 0 to 10 parts by mass of triethyl citrate based on 100 parts by mass of the enteric coating layer formed from the composition for an enteric coating.
(16) The method according to (11) or (12), wherein the composition for an enteric coating comprises 50 to 100 parts by mass of one or more selected from the group consisting of organic acid ester of hydroxypropylmethylcellulose, carboxymethylethylcellulose, and cellulose acetate phthalate and 0 to 10 parts by mass of triethyl citrate based on 100 parts by mass of the enteric coating layer formed from the composition for an enteric coating.
(17) The method according to any one of (11) to (13) and (15), wherein the methacrylic acid-based polymer is a copolymer of methacrylic acid and ethyl acrylate and/or a copolymer of methacrylic acid and methyl methacrylate.
(18) The method according to any one of (11), (12), (14) and (16), wherein the organic acid ester of hydroxypropylmethylcellulose is hydroxypropylmethylcellulose acetate succinate or hydroxypropylmethylcellulose phthalate.
(19) The method according to any one of (11) to (18), wherein the composition for subcoating comprises hydroxypropylcellulose.

### Effect of the Invention

The capsule of the present invention exhibits acid resistance and has enteric properties, and the capsule of the present invention containing a drug, a quasi-drug, or the like as the content can allow the content to dissolve in the intestines. The capsule of the present invention has a preventing action on inactivation of or decrease in efficacy of the ingredient contained by gastric juices, a preventing action on stimulation to the gastric mucosa by the ingredient contained, and a preventing action on inhibition of digestion action of the stomach.

### Brief Description of Drawings

[Figure 1] Figure 1 is a graph illustrating the dissolution properties of acetaminophen from the capsule according to Example 1.
[Figure 2] Figure 2 is a graph illustrating the dissolution properties of acetaminophen from the capsule according to Example 2.
[Figure 3] Figure 3 is a graph illustrating the dissolution properties of acetaminophen from the capsule according to Example 3.
[Figure 4] Figure 4 is a graph illustrating the dissolution properties of acetaminophen from the capsule according to Example 4.

### Mode of Carrying Out the Invention

### (Capsule)

The enteric capsule of the present invention is not particularly limited as long as it comprises: a seamless capsule consisting of a capsule fill and a shell layer; and an enteric coating layer on the shell layer, the enteric coating layer comprising one or more selected from the group consisting of a methacrylic acid-based polymer, polyvinyl acetate phthalate, organic acid ester of hydroxypropylmethylcellulose, carboxymethylethylcellulose, and cellulose acetate phthalate. The enteric capsule of the present invention may further have a subcoating layer between the shell layer of the seamless capsule and the enteric coating layer. The method for manufacturing an enteric capsule of the present invention may comprises coating a seamless capsule consisting of a capsule fill and a shell layer with a composition for an enteric coating to form an enteric coating layer on the shell layer of the seamless capsule, the composition comprising one or more selected from the group consisting of a methacrylic acid-based polymer, polyvinyl acetate phthalate, organic acid ester of hydroxypropylmethylcellulose, carboxymethylethylcellulose, and cellulose acetate phthalate. In cases where the enteric capsule of the present invention further has a subcoating layer between the shell layer of the enteric seamless capsule of the present invention and the enteric coating layer, the method for manufacturing an enteric capsule of the present invention may comprise coating a seamless capsule consisting of a capsule fill and a shell layer with a composition for a subcoating to form a subcoating layer; and then coating the subcoating layer with a composition for an enteric coating to form an enteric coating layer. "Shell" herein means an envelope in which a capsule fill is enveloped.

The enteric capsule of the present invention has an enteric coating layer comprising one or more selected from the group consisting of a methacrylic acid-based polymer, polyvinyl acetate phthalate, organic acid ester of hydroxypropylmethylcellulose, carboxymethylethylcellulose, and cellulose acetate phthalate, and therefore, the capsule fill is not dissolved in the stomach, in which the pH is acidic, but is dissolved in the intestine. The pH at which the capsule fill of the present invention is dissolved is pH of 5 or more, and it is preferably pH of 6 or more for the small intestine as a target, and preferably pH of 7 or more for the large intestine as a target.

### (Seamless capsule)

The seamless capsule is not particularly limited as long as it is a seamless capsule consisting of a capsule fill and a shell layer and has a surface which can be coated with a composition for an enteric coating comprising one or more selected from the group consisting of methacrylic acid-based polymer, polyvinyl acetate phthalate, organic acid ester of hydroxypropylmethylcellulose, carboxymethylethylcellulose, and cellulose acetate phthalate. The capsule fill is not particularly limited as long as it can be filled into a seamless capsule, and examples thereof include a drug and a quasi-drug. The form of the content may be solid, gel, solution, suspension, and emulsion, or paste.

The mass ratio between the capsule fill and the shell layer is preferably in a range of 1:1 to 10:1, and more preferably in a range of 1:1 to 5:1. The particle diameter of the seamless capsule is usually 1 to 15 mm and preferably 2 to 10 mm.

The shell layer may be any of those usually used as a shell of a seamless capsule, and examples thereof include a shell layer containing gelatin, casein, zein, pectin and its derivatives, alginic acid or salt thereof, agar, tragacanth gum, guar gum, locust bean gum, carrageenan, tamarind, mannan, hemilose, starch, chitosan, or the like as a base. The shell layer may contain a plasticizer such as glycerin, propylene glycol, and polyethylene glycol in addition to the base. Among these shell layers, a shell layer containing gelatin and glycerin is preferable. The mass ratio between gelatin and glycerin is preferably in a range of 1:1 to 10:1, and more preferably in a range of 1:1 to 5:1.

Further, the shell layer may contain additives such as a natural dye, a synthetic dye, various sweeteners, a preservative, a water activity-lowering agent, and a pH adjusting agent.

Examples of gelatin include those produced by processing skins, bones, tendons, or the like of cattle, pigs, chickens, fish, or the like as a raw material with acid or alkali to thereby obtain crude collagen and subjecting the crude collagen to thermal extraction. Other examples of gelatin include hydrolyzates and enzyme decomposition products of gelatin and modified gelatin such as succinated gelatin and phthalated gelatin.

As the seamless capsule, a commercially available product thereof can be used, and a seamless capsule also can be manufactured according to a known method for manufacturing a seamless capsule. Examples of the manufacturing method include a dropping method, specifically, for example, a manufacturing method involving allowing a liquid flow to jet from a double nozzle into a coagulating liquid to envelop the capsule fill in the shell.

### (Methacrylic acid-based polymer)

Examples of the methacrylic acid-based polymer include a polymer and copolymer of acrylate and/or methacrylate, and the methacrylic acid-based polymer may be those that can be used as an enteric substance for capsules. Examples thereof include a terpolymer of methyl methacrylate, butyl methacrylate, and dimethylaminoethyl methacrylate; a copolymer of methacrylic acid and methyl methacrylate; a copolymer of methacrylic acid and ethyl acrylate; a terpolymer of ethyl acrylate, methyl methacrylate, and trimethylammmonioethyl methacrylate chloride; and a copolymer of ethyl acrylate and methyl methacrylate. Among these methacrylic acid-based polymers, a copolymer of methacrylic acid and ethyl acrylate and a copolymer of methacrylic acid and methyl methacrylate are preferable; a copolymer of methacrylic acid and ethyl acrylate (component ratio = 1:1), a copolymer of methacrylic acid and methyl methacrylate (component ratio = 1:1), and a copolymer of methacrylic acid and methyl methacrylate (component ratio = 1:2) are more preferable. These methacrylic acid-based polymers may be used singly or in combinations of two or more thereof.

The methacrylic acid-based polymer can be obtained by subjecting acrylate and/or methacrylate as the monomer to a synthesis procedure such as suspension polymerization, bulk polymerization, or solution polymerization, and a commercially available product thereof also can be used. Examples of such a commercially available product of the methacrylic acid-based polymer include EUDRAGIT (registered trademark). Specifically, as for EUDRAGIT, the commercially available product of EUDRAGIT L100-55 can be used as the copolymer of methacrylic acid and ethyl acrylate (component ratio = 1:1); the commercially available product of EUDRAGIT L100 can be used as the copolymer of methacrylic acid and methyl methacrylate (component ratio = 1:1); and the commercially available product of EUDRAGIT S100 can be used as the copolymer of methacrylic acid and methyl methacrylate (component ratio = 1:2) .

### (Polyvinyl acetate phthalate)

Examples of a commercially available product of polyvinyl acetate phthalate include "OPADRY(R) ENTERIC 91 series", and "SURETERIC(R)", which is a raw material that has been blended with other components such as a plasticizer, both manufactured by Colorcon, Inc.

### (Organic acid ester of hydroxypropylmethylcellulose)

Examples of the organic acid ester of hydroxypropylmethylcellulose include hydroxypropylmethylcellulose acetate succinate and hydroxypropylmethylcellulose phthalate. Examples of a commercially available product of such organic acid ester of hydroxypropyl methylcellulose include "Shin-Etsu AQOAT AS-MG" and "HP-55" manufactured by Shin-Etsu Chemical Co., Ltd.

### (Carboxymethylethylcellulose)

Examples of a commercially available product of carboxymethylethylcellulose include "CMEC(R)" manufactured by FREUND CORPORATION.

### (Cellulose acetate phthalate)

Examples of a commercially available product of cellulose acetate phthalate include "Cellacefate" manufactured by Wako Pure Chemical Industries, Ltd.

### (Enteric coating layer)

The enteric coating layer refers to a layer comprising the methacrylic acid-based polymer, polyvinyl acetate phthalate, organic acid ester of hydroxypropylmethylcellulose, carboxymethylethylcellulose, or cellulose acetate phthalate and overlaid on the shell layer of the seamless capsule. The whole of the shell of the seamless capsule is covered with such an enteric coating layer, and the enteric coating layer has enteric properties. The enteric coating layer may be constituted only of a methacrylic acid-based polymer, polyvinyl acetate phthalate, organic acid ester of hydroxypropylmethylcellulose, carboxymethylethylcellulose, or cellulose acetate phthalate, or also may include additives such as a plasticizer, an excipient, a binder, a disintegrator, a lubricant, a surfactant, a solubilizer, a stabilizer, an adsorbent, and a sweetener. Among these additives, a plasticizer and an excipient are preferably included.

In the enteric coating layer, a methacrylic acid-based polymer, polyvinyl acetate phthalate, organic acid ester of hydroxypropylmethylcellulose, carboxymethylethylcellulose, or cellulose acetate phthalate may be included in an amount of 1 to 100 parts by mass based on 100 parts by mass of the enteric coating layer. The composition of the enteric coating layer preferably consists of 30 to 80 parts by mass of a methacrylic acid-based polymer, polyvinyl acetate phthalate, or cellulose acetate phthalate or 50 to 100 parts by mass of organic acid ester of hydroxypropylmethylcellulose, carboxymethylethylcellulose, or cellulose acetate phthalate; 0 to 10 parts by mass of a plasticizer; and 0 to 35 parts by mass of an excipient.

The plasticizer may be any of plasticizers that do not inhibit enteric properties of the enteric coating layer, and examples thereof include triethyl citrate, triacetin, tributyl sebacate, and polyethyl glycol. Among these plasticizers, triethyl citrate is preferable. The excipient may be any of excipients that do not inhibit enteric properties of the enteric coating layer, and examples thereof include talc, aerosil, and sodium aluminum silicate. Among these excipients, talc is preferable.

### (Subcoating layer)

The subcoating layer is for easily forming the enteric coating layer on the shell layer of the seamless capsule. The component contained in the subcoating layer is not particularly limited as long as it helps to form the enteric coating layer on the shell layer of the seamless capsule and also does not inhibit enteric properties of the enteric coating layer. Examples thereof include povidone, cellulose, methylcellulose, hydroxymethylcellulose, calcium carboxymethylcellulose, sodium carboxymethylcellulose, hydroxypropylcellulose, hydroxypropylmethylcellulose phthalate, amorphous cellulose, polypropylpyrrolidone, gelatin, gum arabic, gum acacia, polyethylene glycol, starch, sugar (sucrose, kaolin, dextrose, lactose, etc.), cholesterol, tragacanth, stearic acid, gelatin, casein, lecithin, cetostearyl alcohol, cetyl alcohol, cetyl esters wax, dextrates, dextrin, glyceryl monooleate, glyceryl monostearate, glyceryl palmitostearate, polyoxyethylene alkyl ester, polyoxyethylene castor oil derivative, polyoxyethylene stearate, and polyvinyl alcohol. Among these, hydroxypropylcellulose is preferable. A preferable example of the subcoating layer in the present invention may be a subcoating layer consisting of hydroxypropylcellulose.

Hydroxypropylcellulose described above is not particularly limited as long as it is a cellulose derivative obtained by reacting cellulose and propylene oxide, and examples thereof include hydroxypropylcellulose having a number average molecular weight (Mn) of 40,000 (Hydroxypropylcellulose SSL), hydroxypropylcellulose having a number average molecular weight (Mn) of 100,000 (Hydroxypropylcellulose SL), hydroxypropylcellulose having a number average molecular weight (Mn) of 140,000 (Hydroxypropylcellulose L), hydroxypropylcellulose having a number average molecular weight (Mn) of 620,000 (Hydroxypropylcellulose M), and hydroxypropylcellulose having a number average molecular weight (Mn) of 910,000 (Hydroxypropylcellulose H) . Among these hydroxypropylcelluloses, hydroxypropylcellulose having a number average molecular weight (Mn) of 40,000 (Hydroxypropylcellulose SSL) is preferable. These hydroxypropylcelluloses may be used singly or in combinations of two or more thereof.

### (Manufacturing method)

Examples of the coating method with the composition for an enteric coating or the composition for a subcoating in the manufacturing method include pan coatings and fluid bed coatings, which are coating methods in which a seamless capsule is coated in a coating pan and a fluid bed apparatus respectively. Among these coating methods, pan coating is preferable.

Examples of the composition for an enteric coating include a composition comprising one or more selected from the group consisting of a methacrylic acid-based polymer, polyvinyl acetate phthalate, organic acid ester of hydroxypropylmethylcellulose, carboxymethylethylcellulose, and cellulose acetate phthalate for coating a seamless capsule to form an enteric layer. In addition to a methacrylic acid-based polymer, polyvinyl acetate phthalate, organic acid ester of hydroxypropylmethylcellulose, carboxymethylethylcellulose, or cellulose acetate phthalate, the composition for an enteric coating may include additives mentioned above such as a plasticizer, an excipient, a binder, a disintegrator, a lubricant, a surfactant, a solubilizer, a stabilizer, an adsorbent, and a sweetener. In order to facilitate coating operation for the seamless capsule, the composition preferably further include a solvent, and examples of such a solvent include alcohols such as ethanol and isopropanol, ethers such as diethyl ether, and acetone. Among these solvents, alcohols are preferable, and ethanol is more preferable. Examples of the form of the composition for an enteric coating include solid, gel, solution, suspension, emulsion, and paste, and solution is preferable.

Upon coating a seamless capsule, the seamless capsule is coated with the coating composition for an enteric coating such that the amount of a methacrylic acid-based polymer, polyvinyl acetate phthalate, organic acid ester of hydroxypropylmethylcellulose, carboxymethylethylcellulose, or cellulose acetate phthalate from the composition is 1.0 to 10 mg/cm² in terms of solids, preferably such that the amount of a methacrylic acid-based polymer is 2.0 to 6.0 mg/cm² or that the amount of polyvinyl acetate phthalate, organic acid ester of hydroxypropylmethylcellulose, carboxymethylethylcellulose, or cellulose acetate phthalate from the composition is 6.0 to 10 mg/cm².

The composition for a subcoating refers to a composition for forming a subcoating layer with which a seamless capsule is coated to easily form the enteric coating layer on the shell layer. The composition for a subcoating comprises the components mentioned above to favorably form an enteric coating layer on the shell layer of the seamless capsule. In order to facilitate coating operation for a seamless capsule, the composition preferably further include a solvent, and examples of such a solvent include alcohols such as ethanol and isopropanol, ethers such as diethyl ether, and acetone. Among these solvents, alcohols are preferable, and ethanol is more preferable. Examples of the form of the composition for a subcoating include solid, gel, solution, suspension, emulsion, and paste, and solution is preferable.

### (Rotary capsule)

Mentions have been made for a seamless capsule supra, but the same also can be applied to a soft capsule manufactured with a rotary type apparatus for manufacturing capsules (hereinafter referred to as "rotary capsule") according to a method involving forming a subcoating layer and then coating a capsule with a composition for an enteric coating, provided that methods involving coating a capsule with a composition for an enteric coating without forming a subcoating layer are excluded. Specifically, kinds and materials of the capsule fill and the shell layer, the mass ratio between the capsule fill and the shell layer, and kinds and materials of the subcoating layer and the enteric coating layer, and coating methods are the same as for the seamless capsule described above.

As the rotary capsule, commercially available products can be used, and the rotary capsule also can be manufactured according to a known method for manufacturing rotary capsules. Examples of such manufacturing methods include methods involving a stamping process using a soft capsules-filling machine of rotary die type or the like, or plating process. The soft capsules-filling machine of rotary die type uses a method in which two shell sheets formed by spreading a liquid for soft capsule shells on a rotating drum are stamped into a shape of a capsule with a pair of rotating dies (die rolls), and in the method, forming a soft capsule and filling a capsule fill are carried out simultaneously. In cases of rotary capsules, the method involving forming a subcoating layer and then coating with a composition for an enteric coating is particularly preferable.

The shape of the rotary capsule is not particularly limited as long as the shape is suitable for oral administration. Examples thereof include an oval (football) shape, an oblong (ellipse) shape, a round (sphere) shape, and special shapes such as a tube shape.

### Examples

The present invention will be described specifically below by way of examples, but the present invention is not limited thereto.

The following were used as raw materials.
Gelatin: acid-processed pigskin gelatin (manufactured by Nippi. Inc.)
Glycerin: (manufactured by Sakamoto Yakuhin kogyo Co., Ltd.)
Acetaminophen: (manufactured by Yamamoto Chemical Industry K.K.)
MCT (MYRITOL 318): (manufactured by BASF SE)
Hydroxypropylcellulose-SSL: (manufactured by EVONIK)
Methacrylic acid-based polymer: EUDRAGIT L100-55, EUDRAGIT L100, EUDRAGIT S100 (manufactured by EVONIK)
Polyvinyl acetate phthalate (SURETERIC): (manufactured by Colorcon, Inc.)
Hydroxypropylmethylcellulose acetate succinate (Shin-Etsu AQOAT AS-MG): (manufactured by Shin-Etsu Chemical Co., Ltd.)
Hydroxypropylmethylcellulose phthalate (HP-55): (manufactured by Shin-Etsu Chemical Co., Ltd.)

### (Manufacture of enteric capsule)

### 1. Manufacture of seamless capsule

The materials were mixed and stirred in the mass percent (%) shown in Table 1 and the resultant mixture was passed through a plain-woven wire netting of 80 mesh to obtain a capsule fill. The materials in parts by mass shown in Table 2 were mixed to obtain liquid for a shell. A seamless capsule was manufactured from the capsule fill and the liquid for a shell using an apparatus for manufacturing seamless capsules manufactured by Fuji Capsule CO., LTD. according to a seamless dropping method. The seamless capsule has a mass of the content of 100 mg, a mass of the shell of about 25 mg, and a particle diameter of about 6 mm.

**Table 1 Formulation of capsule fill**

| Materials | Mass percent (%) |
|---|---|
| Acetaminophen | 2 |
| MCT (MYRITOL 318) | 98 |
| Total | 100 |

**Table 2 Formulation of liquid for shell**

| Materials | Parts by mass |
|---|---|
| Acid-processed pigskin gelatin | 100 |
| Glycerin | 50 |

### 2. Coating step

The coating in Examples 1 to 4 was conducted by pan coating using "HC-Lab" manufactured by FREUND CORPORATION according to the following procedure.

### [Examples 1 to 3]

250 g of the seamless capsule described above was coated for 43 to 48 minutes under a condition of a temperature of air fed of 50 to 55°C by spraying the composition for a subcoating at a rate of 1.2 to 3.6 g/min. Then, the resulting seamless capsule was dried at 45°C for 10 minutes to obtain a subcoated capsule sample. The subcoated capsule sample obtained was further coated under a condition of a temperature of air fed of 32 to 45°C for 57 to 59 minutes by spraying the composition for an enteric coating at a rate of 1.2 to 3.0 g/min so that the amount of the methacrylic acid-based polymer in the composition was 4.0 mg/cm² in terms of solids. Then, the resulting capsule sample was dried at room temperature for 10 minutes to manufacture an enteric capsule. The mass and ratio of the seamless capsule and each component per enteric capsule are shown in Table 4.

### [Example 4]

250 g of the seamless capsule described above was coated under a condition of a temperature of air fed of 34 to 36°C for 57 minutes by spraying the composition for an enteric coating at a rate of 1.4 to 2.7 g/min so that the amount of the methacrylic acid-based polymer in the composition was 4.0 mg/cm² in terms of solids. Then, the resulting seamless capsule was dried at room temperature for 10 minutes to manufacture an enteric capsule. The mass and ratio of the seamless capsule and each component per enteric capsule are shown in Table 4.

The coating in Examples 5 to 8 was conducted by pan coating using "PRC-05" manufactured by Powrex corp. according to the following procedure.

### [Example 5]

250 g of the seamless capsule described above was coated under a condition of a temperature of air fed of 50 to 55°C for 62 minutes by spraying the composition for a subcoating at a rate of 1.1 to 2.9 g/min. Then, the resulting seamless capsule was dried at 45°C for 10 minutes to obtain a subcoated capsule sample. The subcoated capsule sample obtained was further coated under a condition of a temperature of air fed of 45 to 55°C for 67 minutes by spraying the composition for an enteric coating at a rate of 1.0 to 2.8 g/min so that the amount of polyvinyl acetate phthalate in the composition was 9.2 mg/cm² in terms of solids. Then, the resulting capsule sample was dried at room temperature for 10 minutes to manufacture an enteric capsule. The mass and ratio of the seamless capsule and each component per enteric capsule are shown in Table 4.

### [Examples 6 to 8]

250 g of the seamless capsule described above was coated under a condition of a temperature of air fed of 50 to 55°C for 59 to 65 minutes by spraying the composition for a subcoating at a rate of 1.1 to 3.4 g/min. Then, the resulting seamless capsule was dried at 45°C for 10 minutes to obtain a subcoated capsule sample. The subcoated capsule sample obtained was further coated under a condition of a temperature of air fed of 34 to 42°C for 100 to 130 minutes by spraying the composition for an enteric coating at a rate of 1.2 to 3.6 g/min so that the amount of hydroxypropylmethylcellulose acetate succinate and hydroxypropylmethylcellulose phthalate from the composition was 9.2 mg/cm² in terms of solids. Then, the resulting capsule sample was dried at room temperature for 10 minutes to manufacture an enteric capsule. The mass and ratio of the seamless capsule and each component per enteric capsule are shown in Table 4.

**Table 3 Formulations of composition for subcoating and composition for enteric coating**

| | Materials | Example 1 (g) | Example 2 (g) | Example 3 (g) | Example 4 (g) |
|---|---|---|---|---|---|
| Composition for subcoating | HPC-SSL | 8.8 | 8.8 | 8.8 | - |
| | 95% Ethanol | 101.1 | 101.1 | 101.1 | - |
| Composition for enteric coating | EUDRAGIT L100-55 | 8.8 | - | - | 8.8 |
| | EUDRAGIT L100 | - | 8.8 | - | - |
| | EUDRAGIT S100 | - | - | 8.8 | - |
| | Triethyl citrate | 0.9 | 0.9 | 0.9 | 0.9 |
| | Talc | 4.4 | 4.4 | 4.4 | 4.4 |
| | 95% Ethanol | 126.9 | 126.9 | 126.9 | 126.9 |
| | | | | | |

| | Materials | Example 5 (g) | Example 6 (g) | Example 7 (g) | Example 8 (g) |
|---|---|---|---|---|---|
| Composition for subcoating | HPC-SSL | 8.8 | 8.8 | 8.8 | 8.8 |
| | 95% Ethanol | 101.1 | 101.1 | 101.1 | 101.1 |
| Composition for enteric coating | SURETERIC | 21.1 | - | - | - |
| | Shin-Etsu AQOAT AS-MG | - | 8.5 | - | 8.5 |
| | HPMCP HP-55 | - | - | 11.3 | - |
| | Triethyl citrate | - | - | 1.1 | 0.55 |
| | 80% Ethanol | - | 132.5 | 128.6 | 132.5 |
| | 90% Ethanol | 119.9 | - | - | |

**Table 4 Mass of seamless capsule and each component per capsule**

| Materials | Example 1 (mg) | Example 2 (mg) | Example 3 (mg) | Example 4 (mg) |
|---|---|---|---|---|
| Seamless capsule | 125.0 | 125.0 | 125.0 | 125.0 |
| HPC-SSL | 4.5 | 4.5 | 4.5 | - |
| EUDRAGIT L100-55 | 4.5 | - | - | 4.5 |
| EUDRAGIT L100 | - | 4.5 | - | - |
| EUDRAGIT S100 | - | - | 4.5 | - |
| Triethyl citrate | 0.5 | 0.5 | 0.5 | 0.5 |
| Talc | 2.3 | 2.3 | 2.3 | 2.3 |
| Total | 136.8 | 136.8 | 136.8 | 132.3 |
| | | | | |

| Materials | Example 5 (mg) | Example 6 (mg) | Example 7 (mg) | Example 8 (mg) |
|---|---|---|---|---|
| Seamless capsule | 125.0 | 125.0 | 125.0 | 125.0 |
| HPC-SSL | 4.5 | 4.5 | 4.5 | 4.5 |
| SURETERIC | 10.4 | - | - | - |
| Shin-Etsu AQOAT AS-MG | - | 10.4 | - | 10.4 |
| HPMCP HP-55 | - | - | 10.4 | - |
| Triethyl citrate | - | - | 1.0 | 0.5 |
| Total | 139.9 | 139.9 | 140.9 | 140.4 |

### 3. Dissolution test

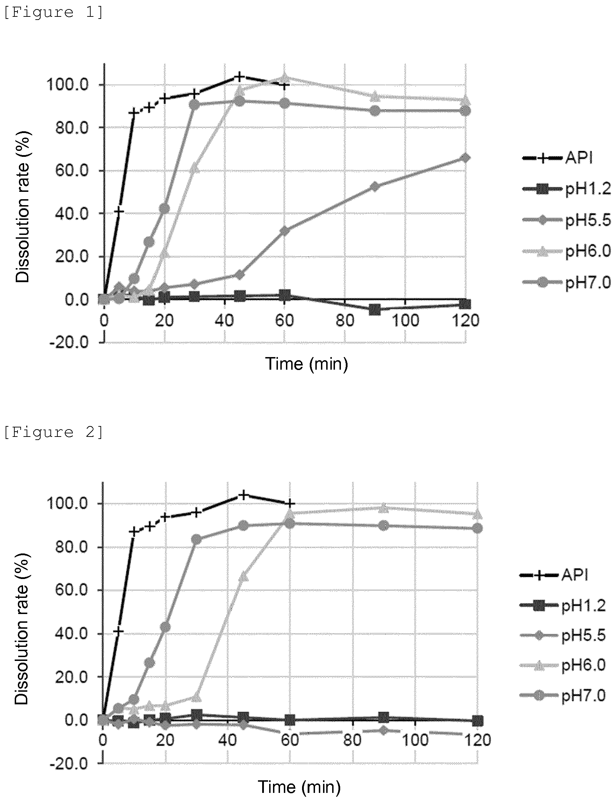

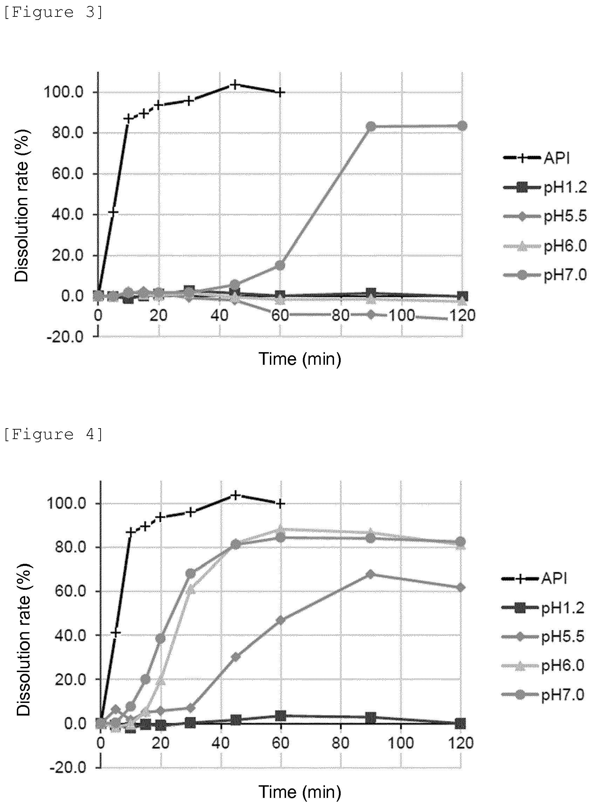

Each of the enteric capsules according to Examples 1 to 4 was checked for acid resistance for 2 hours according to the method of dissolution test prescribed in Japanese pharmacopeia, 16th edition, using the first fluid for dissolution test in Japanese pharmacopeia under conditions shown below. The dissolution test was carried out over 2 hours using McIlvaine buffer solution diluted to pH 5.5, pH 6.0, or pH 7.0, to evaluate dissolution properties of acetaminophen (API) from the enteric capsule at each pH. The results of the dissolution test are shown in Figures 1 to 4.

### [Conditions]

Method of dissolution test: Paddle method for dissolution test in Japanese pharmacopeia
Number of rotation of paddle: 50 rpm
Dissolution tester: NTR-6100 (manufactured by Toyama Sangyo Co., Ltd.)
UV detector: V-630 (manufactured by JASCO Corporation) *: "API" means dissolution properties of only the active pharmaceutical ingredient.

From the results of the dissolution test, it is clear that the capsules according to Examples 1 to 4 each exhibit acid resistance and have enteric properties. In more detail, it can be seen that dissolution properties in Examples 1 to 4 each show a characteristic dissolution curve. In Example 1, dissolution almost did not occur at pH 1.2 but occurred at pH 5.5 or more, and therefore it is expected that dissolution occurs in the duodenum when being taken in the body. In Example 2, dissolution almost did not occur at pH 1.2 and pH 5.5 but occurred at pH 6.0 or more, and therefore it is expected that dissolution occurs in the small intestine when being taken in the body. In Example 3, dissolution almost did not occur at pH 6.0 or less but occurred after a certain holding time at pH 7.0, and therefore it is expected that dissolution occurs in the large intestine when being taken in the body. In Example 4, a behavior of dissolution similar to that in Example 1 was observed, and therefore it is expected that dissolution occurs in the duodenum when being taken in the body.

It was also found that though the methacrylic acid-based polymer as an enteric substance in the shell of the capsule accounts for a moderate proportion in the components constituting the shell, it can retain enteric properties of the capsule.

### 4. Disintegration test

Each of the enteric capsules according to Examples 5 to 8 was checked for acid resistance for 2 hours according to the method of disintegration test prescribed in Japanese pharmacopeia, 16th edition, using the first fluid for disintegration test in Japanese pharmacopeia under conditions shown below. All six samples for each Examples 5 to 8 did not broken to retain the original state without aperture or breakage of the capsule shell.

Furthermore, similarly, new enteric capsule samples according to Examples 5 to 8 were provided and subjected to a disintegration test over 60 minutes using the second fluid for disintegration test in Japanese pharmacopeia. The basket-rack assembly was taken from the test fluid, and when the state of disintegration of the capsule was observed, any residue was not found in the glass tube.

**Table 5 Results of disintegration test**

| | Example 5 | Example 6 | Example 7 | Example 8 |
|---|---|---|---|---|
| 1st fluid for disintegration test in Japanese Pharmacopoeia (pH 1.2) | Compliant | Compliant | Compliant | Compliant |
| 2nd fluid for disintegration test in Japanese Pharmacopoeia (pH 6.8) | Compliant | Compliant | Compliant | Compliant |

### [Conditions]

Method of disintegration test: Disintegration test in Japanese pharmacopoeia enteric coated tablets and capsules
Dissolution tester: NT-40H (manufactured by Toyama Sangyo Co., Ltd.)

### (Manufacture of enteric capsule)

### 1. Manufacture of rotary capsule

A soft capsule was manufactured from the capsule fill shown in Table 6 and a liquid for a shell obtained by mixing the materials in the amounts in parts by mass shown in Table 7 using a rotary type apparatus for manufacturing capsules manufactured by Fuji Capsule CO., LTD. according to a stamping process. The soft capsule was of OVAL5 and had a mass of the content of 280 mg, a mass of the shell of about 170 mg, a major axis of about 13 mm, and a minor axis of about 7.5 mm.

**Table 6 Formulation of capsule fill**

| Materials | Mass percent (%) |
|---|---|
| MCT | 100 |
| Total | 100 |

**Table 7 Formulation of liquid for shell**

| Materials | Parts by mass |
|---|---|
| Gelatin | 100 |
| Glycerin | 35 |

### 2. Coating step

The coating was conducted by pan coating using "PRC-05" manufactured by Powrex corp. according to the following procedure.

250 g of the rotary capsule described above was coated under a condition of a temperature of air fed of 50 to 55°C by spraying the composition for a subcoating shown in Table 8 at a rate of 1.2 to 3.6 g/min so that the amount of the composition for a subcoating was as shown in Table 9 in terms of solids. Then, the resulting rotary capsule was dried at 45°C for 10 minutes to obtain a subcoated capsule sample. Each of the subcoated capsule samples obtained was further coated under a condition of a temperature of air fed of 32 to 45°C by spraying the composition for an enteric coating shown in Table 8 at a rate of 1.2 to 3.0 g/min so that the amount of the composition for an enteric composition was as shown in Table 10 in terms of solids. Then, the resulting capsule sample was dried at room temperature for 10 minutes to manufacture an enteric capsule.

**Table 8 Formulations of composition for subcoating and composition for enteric coating**

| | Materials | | S-1(g) | S-2(g) |
|---|---|---|---|---|
| Composition for subcoating | HPC-SSL | | 8.8 | - |
| | HPMC | | - | 8.8 |
| | 95% Ethanol | | 101.1 | 101.1 |

| | Materials | E-1(g) | E-2(g) | E-3(g) |
|---|---|---|---|---|
| Composition for enteric coating | EUDRAGIT L100-55 | 8.8 | - | - |
| | EUDRAGIT L100 | - | 8.8 | |
| | EUDRAGIT S100 | - | - | 8.8 |
| | Triethyl citrate | 0.9 | 0.9 | 0.9 |
| | Talc | 4.4 | 4.4 | 4.4 |
| | 95% Ethanol | 126.9 | 126.9 | 126.9 |

**Table 9 Mass of rotary capsule and each component of composition for subcoating per capsule and amount of composition for subcoating per unit area (in terms of solids)**

| Materials | Case 1 | Case 2 | Case 3 | Case 4 | Case 5 |
|---|---|---|---|---|---|
| Composition for subcoating used | - | S-1 | S-1 | S-1 | S-1 |
| Rotary capsule (mg) | 450.0 | 450.0 | 450.0 | 450.0 | 450.0 |
| HPC-SSL (mg) | - | 2.7 | 5.4 | 8.0 | 16.0 |
| HPMC (mg) | - | - | - | - | - |
| Total (mg) | 450.0 | 452.7 | 455.4 | 458.0 | 466.0 |
| Amount of composition for subcoating per unit area (in terms of solids) (mg/cm²) | 0 | 1.0 | 2.0 | 3.0 | 6.0 |
| | | | | | |

| Materials | Case 6 | Case 7 | Case 8 | Case 9 | |
|---|---|---|---|---|---|
| Composition for subcoating used | S-1 | S-1 | S-2 | S-2 | |
| Rotary capsule (mg) | 450.0 | 450.0 | 450.0 | 450.0 | |
| HPC-SSL (mg) | 24.1 | 32.1 | - | - | |
| HPMC (mg) | - | - | 5.4 | 8.0 | |
| Total (mg) | 474.1 | 482.1 | 455.4 | 458.0 | |
| Amount of composition for subcoating per unit area (in terms of solids) (mg/cm²) | 9.0 | 12.0 | 2.0 | 3.0 | |

**Table 10 Mass of each component of composition for enteric coating per capsule and amount of methacrylic acid-based polymer in composition for enteric coating per unit area (in terms of solids)**

| Materials | Case A | Case B | Case C | Case D | Case E |
|---|---|---|---|---|---|
| Composition for enteric coating used | E-1 | E-1 | E-1 | E-2 | E-3 |
| EUDRAGIT L100-55 (mg) | 5.4 | 10.7 | 16.1 | - | - |
| EUDRAGIT L100 (mg) | - | - | - | 10.7 | - |
| EUDRAGIT S100 (mg) | - | - | - | - | 10.7 |
| Triethyl citrate (mg) | 0.5 | 1.1 | 1.6 | 1.1 | 1.1 |
| Talc (mg) | 2.7 | 5.4 | 8.0 | 5.4 | 5.4 |
| Total (mg) | 8.6 | 17.2 | 25.7 | 17.2 | 17.2 |
| Amount of methacrylic acid-based polymer in composition for enteric coating per unit area (in terms of solids) (mg/cm²) | 2.0 | 4.0 | 6.0 | 4.0 | 4.0 |

### 3. Disintegration test (acid resistance test)

Each of the enteric capsules was checked for acid resistance for 2 hours according to the method of disintegration test prescribed in Japanese pharmacopeia, 16th edition, using the first fluid for disintegration test (pH 1.2) in Japanese pharmacopeia under conditions shown below. The results are shown in Table 11.

**Table 11 Results of disintegration test**

| | | HPC-SSL | | | | | | |
|---|---|---|---|---|---|---|---|---|
| | | Case 1 | Case 2 | Case 3 | Case 4 | Case 5 | Case 6 | Case 7 |
| EUDRAGI T L100-55 | Cas e A | Noncomplia nt | Noncomplia nt | Noncomplia nt | Noncomplia nt | Noncomplia nt | Noncomplia nt | Noncomplia nt |
| | Cas e B | Noncomplia nt | Noncomplia nt | Noncomplia nt | Compliant | Compliant | Compliant | Compliant |
| | Cas e C | Noncomplia nt | Noncomplia nt | Noncomplia nt | Compliant | Compliant | Compliant | Compliant |
| EUDRAGI T L100 | Cas e D | - | - | Noncomplia nt | Compliant | - | - | - |
| EUDRAGI TS100 | Cas e E | - | - | Noncomplia nt | Compliant | - | - | - |
| | | | | | | | | |

| | | HPMC | | | | | | |
|---|---|---|---|---|---|---|---|---|
| | | | | Case 8 | Case 9 | | | |
| EUDRAGIT L100-55 | Case A | | | Noncompliant | Noncompliant | | | |
| | Case B | | | Noncompliant | Compliant | | | |
| | Case C | | | Noncompliant | Compliant | | | |
| EUDRAGIT L100 | Case D | | | Noncompliant | Compliant | | | |
| EUDRAGIT S100 | Case E | | | Noncompliant | Compliant | | | |

### [Conditions]

Method of disintegration test: Disintegration test in Japanese pharmacopeia
Disintegration tester: NT-40H (manufactured by Toyama Sangyo Co., Ltd.)

From the results of the disintegration test, enteric capsules exhibited acid resistance under conditions of 3.0 mg/cm² or more of a subcoating and 4.0 mg/cm² or more of an enteric coating. Under a condition of 2.0 mg/cm² of the enteric coating, an aperture of the capsule was observed within 60 minutes when the amount of the subcoating was 3.0 mg/cm² or less, and an aperture of the capsule was observed within 80 minutes even when the amount of the subcoating was 12.0 mg/cm². Therefore, it seems that under a condition of 2.0 mg/cm² of the enteric coating, the amount of coating is not sufficient to obtain acid resistance. Also under a condition of 4.0 mg/cm² of the enteric coating, an aperture of the capsule was observed within 110 minutes when the amount of the subcoating was 2.0 mg/cm² or less, resulting in no acid resistance. Also under a condition of 6.0 mg/cm² of the enteric coating, an aperture of the capsule was observed within 120 minutes or less when the amount of the subcoating was 2.0 mg/cm² or less, resulting in no acid resistance. The following is presumed: this is because fine unevenness present on the bonded part of the soft capsule prevents formation of the enteric coating layer, and by increasing the amount of the subcoating, the subcoating layer fills unevenness of the bonded part to form a smooth surface structure, which allows a favorable enteric coating.

The following are further embodiments of the present invention.
(1) An enteric capsule comprising:
   a rotary capsule consisting of a capsule fill and a shell layer;
   a subcoating layer on the shell layer of the rotary capsule; and
   an enteric coating layer on the subcoating layer, the enteric coating layer comprising one or more selected from the group consisting of a methacrylic acid-based polymer, polyvinyl acetate phthalate, organic acid ester of hydroxypropylmethylcellulose, carboxymethylethylcellulose, and cellulose acetate phthalate.
(2) The enteric capsule according to (1), wherein the shell layer of the rotary capsule comprises gelatin and glycerin.
(3) The enteric capsule according to (1) or (2), wherein the enteric coating layer comprises 30 to 80 parts by mass of one or more selected from the group consisting of a methacrylic acid-based polymer, polyvinyl acetate phthalate, and cellulose acetate phthalate based on 100 parts by mass of the enteric coating layer.
(4) The enteric capsule according to (1) or (2), wherein the enteric coating layer comprises 50 to 100 parts by mass of one or more selected from the group consisting of organic acid ester of hydroxypropylmethylcellulose, carboxymethylethylcellulose, and cellulose acetate phthalate based on 100 parts by mass of the enteric coating layer.
(5) The enteric capsule according to (1) or (2), wherein the enteric coating layer comprises 30 to 80 parts by mass of one or more selected from the group consisting of a methacrylic acid-based polymer, polyvinyl acetate phthalate, and cellulose acetate phthalate and 0 to 10 parts by mass of triethyl citrate based on 100 parts by mass of the enteric coating layer.
(6) The enteric capsule according to (1) or (2), wherein the enteric coating layer comprises 50 to 100 parts by mass of one or more selected from the group consisting of organic acid ester of hydroxypropylmethylcellulose, carboxymethylethylcellulose, and cellulose acetate phthalate and 0 to 10 parts by mass of triethyl citrate based on 100 parts by mass of the enteric coating layer.
(7) The enteric capsule according to any one of (1) to (3) and (5), wherein the methacrylic acid-based polymer is a copolymer of methacrylic acid and ethyl acrylate and/or a copolymer of methacrylic acid and methyl methacrylate.
(8) The enteric capsule according to any one of (1), (2), (4) and (6), wherein the organic acid ester of hydroxypropylmethylcellulose is hydroxypropylmethylcellulose acetate succinate or hydroxypropylmethylcellulose phthalate.
(9) The enteric capsule according to any one of (1) to (8), wherein the subcoating layer consists of hydroxypropylcellulose.
(10) A method for manufacturing an enteric capsule, comprising:
   coating a rotary capsule consisting of a capsule fill and a shell layer with a composition for a subcoating to form a subcoating layer; and then,
   coating the subcoating layer with a composition for an enteric coating to form an enteric coating layer, the composition comprising one or more selected from the group consisting of a methacrylic acid-based polymer, polyvinyl acetate phthalate, organic acid ester of hydroxypropylmethylcellulose, carboxymethylethylcellulose, and cellulose acetate phthalate.
(11) The method according to (10), wherein the composition for an enteric coating comprises 30 to 80 parts by mass of one or more selected from the group consisting of a methacrylic acid-based polymer, polyvinyl acetate phthalate, and cellulose acetate phthalate based on 100 parts by mass of the enteric coating layer to be formed of the composition for an enteric coating.
(12) The method according to (10), wherein the composition for an enteric coating comprises 50 to 100 parts by mass of one or more selected from the group consisting of organic acid ester of hydroxypropylmethylcellulose, carboxymethylethylcellulose, and cellulose acetate phthalate based on 100 parts by mass of the enteric coating layer to be formed of the composition for an enteric coating.
(13) The method according to (10), wherein the composition for an enteric coating comprises 30 to 80 parts by mass of one or more selected from the group consisting of a methacrylic acid-based polymer, polyvinyl acetate phthalate, and cellulose acetate phthalate and 0 to 10 parts by mass of triethyl citrate based on 100 parts by mass of the enteric coating layer to be formed of the composition for an enteric coating.
(14) The method according to claim (10), wherein the composition for an enteric coating comprises 50 to 100 parts by mass of one or more selected from the group consisting of organic acid ester of hydroxypropylmethylcellulose, carboxymethylethylcellulose, and cellulose acetate phthalate and 0 to 10 parts by mass of triethyl citrate based on 100 parts by mass of the enteric coating layer to be formed of the composition for an enteric coating.
(15) The method according to any one of (10) to (11) and (13), wherein the methacrylic acid-based polymer is a copolymer of methacrylic acid and ethyl acrylate and/or a copolymer of methacrylic acid and methyl methacrylate.
(16) The method according to any one of (10), (12) and (14), wherein the organic acid ester of hydroxypropylmethylcellulose is hydroxypropylmethylcellulose acetate succinate or hydroxypropylmethylcellulose phthalate.
(17) The method according to any one of (10) to (16), wherein the composition for a subcoating comprises hydroxypropylcellulose.

### Industrial Applicability

The capsule has enteric properties and comprises: a seamless capsule consisting of a capsule fill and a shell layer; and a coating layer on the shell layer, the coating layer comprising one or more selected from the group consisting of a methacrylic acid-based polymer, polyvinyl acetate phthalate, organic acid ester of hydroxypropylmethylcellulose, carboxymethylethylcellulose, and cellulose acetate phthalate. According to the capsule, a drug, a quasi-drug, or the like which is inactivated or deteriorated in efficacy by gastric juices or causes stimulation to the gastric mucosa, inhibition of the process of digestion in the stomach, or the like can be prescribed.

## Claims

1. An enteric capsule comprising:
a seamless capsule consisting of a capsule fill and a shell layer; and
an enteric coating layer on the shell layer, the enteric coating layer comprising one or more selected from the group consisting of a methacrylic acid-based polymer, polyvinyl acetate phthalate, organic acid ester of hydroxypropylmethylcellulose, carboxymethylethylcellulose, and cellulose acetate phthalate.

2. The enteric capsule according to claim 1, wherein the shell layer of the seamless capsule comprises gelatin and glycerin.

3. The enteric capsule according to claim 1 or 2, wherein the enteric coating layer comprises 30 to 80 parts by mass of one or more selected from the group consisting of a methacrylic acid-based polymer, polyvinyl acetate phthalate, and cellulose acetate phthalate based on 100 parts by mass of the enteric coating layer.

4. The enteric capsule according to claim 1 or 2, wherein the enteric coating layer comprises 50 to 100 parts by mass of one or more selected from the group consisting of organic acid ester of hydroxypropylmethylcellulose, carboxymethylethylcellulose, and cellulose acetate phthalate based on 100 parts by mass of the enteric coating layer.

5. The enteric capsule according to claim 1 or 2, wherein the enteric coating layer comprises 30 to 80 parts by mass of one or more selected from the group consisting of a methacrylic acid-based polymer, polyvinyl acetate phthalate, and cellulose acetate phthalate and 0 to 10 parts by mass of triethyl citrate based on 100 parts by mass of the enteric coating layer.

6. The enteric capsule according to claim 1 or 2, wherein the enteric coating layer comprises 50 to 100 parts by mass of one or more selected from the group consisting of organic acid ester of hydroxypropylmethylcellulose, carboxymethylethylcellulose, and cellulose acetate phthalate and 0 to 10 parts by mass of triethyl citrate based on 100 parts by mass of the enteric coating layer.

7. The enteric capsule according to any one of claims 1 to 3 and 5, wherein the methacrylic acid-based polymer is a copolymer of methacrylic acid and ethyl acrylate and/or a copolymer of methacrylic acid and methyl methacrylate.

8. The enteric capsule according to any one of claims 1, 2, 4 and 6, wherein the organic acid ester of hydroxypropylmethylcellulose is hydroxypropylmethylcellulose acetate succinate or hydroxypropylmethylcellulose phthalate.

9. The enteric capsule according to any one of claims 1 to 8, further comprising a subcoating layer between the shell layer of the seamless capsule and the enteric coating layer.

10. The enteric capsule according to claim 9, wherein the subcoating layer consists of hydroxypropylcellulose.

11. A method for manufacturing an enteric capsule, comprising:
coating a seamless capsule consisting of a capsule fill and a shell layer with a composition for an enteric coating to form an enteric coating layer, the composition comprising one or more selected from the group consisting of a methacrylic acid-based polymer, polyvinyl acetate phthalate, organic acid ester of hydroxypropylmethylcellulose, carboxymethylethylcellulose, and cellulose acetate phthalate.

12. A method for manufacturing an enteric capsule, comprising:
coating a seamless capsule consisting of a capsule fill and a shell layer with a composition for a subcoating to form a subcoating layer; and then,
coating the subcoating layer with a composition for an enteric coating to form an enteric coating layer, wherein the composition comprising one or more selected from the group consisting of a methacrylic acid-based polymer, polyvinyl acetate phthalate, organic acid ester of hydroxypropylmethylcellulose, carboxymethylethylcellulose, and cellulose acetate phthalate.

13. The method according to claim 11 or 12, wherein the composition for an enteric coating comprises 30 to 80 parts by mass of one or more selected from the group consisting of a methacrylic acid-based polymer, polyvinyl acetate phthalate, and cellulose acetate phthalate based on 100 parts by mass of the enteric coating layer formed from the composition for an enteric coating.

14. The method according to claim 11 or 12, wherein the composition for an enteric coating comprises 50 to 100 parts by mass of one or more selected from the group consisting of organic acid ester of hydroxypropylmethylcellulose, carboxymethylethylcellulose, and cellulose acetate phthalate based on 100 parts by mass of the enteric coating layer formed from the composition for an enteric coating.

15. The method according to claim 11 or 12, wherein the composition for an enteric coating comprises 30 to 80 parts by mass of one or more selected from the group consisting of a methacrylic acid-based polymer, polyvinyl acetate phthalate, and cellulose acetate phthalate and 0 to 10 parts by mass of triethyl citrate based on 100 parts by mass of the enteric coating layer formed from the composition for an enteric coating.

16. The method according to claim 11 or 12, wherein the composition for an enteric coating comprises 50 to 100 parts by mass of one or more selected from the group consisting of organic acid ester of hydroxypropylmethylcellulose, carboxymethylethylcellulose, and cellulose acetate phthalate and 0 to 10 parts by mass of triethyl citrate based on 100 parts by mass of the enteric coating layer formed from the composition for an enteric coating.

17. The method according to any one of claims 11 to 13 and 15, wherein the methacrylic acid-based polymer is a copolymer of methacrylic acid and ethyl acrylate and/or a copolymer of methacrylic acid and methyl methacrylate.

18. The method according to any one of claims 11, 12, 14 and 16, wherein the organic acid ester of hydroxypropylmethylcellulose is hydroxypropylmethylcellulose acetate succinate or hydroxypropylmethylcellulose phthalate.

19. The method according to any one of claims 11 to 18, wherein the composition for subcoating comprises hydroxypropylcellulose.
